# EUROPEAN PATENT SPECIFICATION

(11) **EP 3 184 090 B1**
(45) Date of publication and mention of the grant of the patent: **22.01.2020**
(21) Application number: 15201516.0
(22) Date of filing: 21.12.2015
(51) Int. Cl.: A61F 5/058, A61F 5/01

(54) **MOVEMENT RESTRAINING DEVICE**
BEWEGUNGSRÜCKHALTEVORRICHTUNG
DISPOSITIF DE RESTRICTION DE MOUVEMENT

(43) Date of publication of application: 28.06.2017
(73) Proprietor: 3M Innovative Properties Company, Saint Paul, MN 55133-3427 (US)
(72) Inventor: Graichen, Andreas Heinz, 40721 Hilden (DE)
(74) Representative: Lanoe, Benjamin

(56) References cited:
- US-A- 4 183 098
- US-A- 5 232 436
- US-A- 5 792 077
- US-A1- 2012 144 545
- US-B1- 6 515 593

## Description

The present invention relates to movement restraining devices for medical purposes, for human patients as well as for animals.

Movement of body parts at joints sometimes needs to be restricted when the movement would impede healing or cause damage to the -human or animal - patient. Traditionally, a cast or a splint is applied at the body joint to prevent movement of the body part, when the joint is damaged or has recently been subject to surgery, for example. Similarly, when a catheter is inserted through skin into a blood vessel of a body part in the vicinity of a body joint, movement of the body part can bend the catheter and create a kink, which may lead to a loss of a blood pressure signal or to complete or partial blocking of a catheter tube. It is not unusual for a patient to make uncontrolled movements when waking up from anaesthesia after surgery or to try getting rid of inserted pieces as they are uncomfortable. Such movements of a body part can lead to kinking of a catheter or of catheter tubes, e.g. when a radialis arterial catheter is kinked by inward movements of the hand. Braces have frequently been used to completely block movement, i.e. immobilization, of the body part in order to avoid catheter kinking.

For some types of injuries or deformities it is not desirable to block movement of a body part at a joint completely. Generally, there is a trend towards only partial immobilization to facilitate faster healing. One attempt towards only partial immobilization is described in U.S. patent no. 5'921'945, in which a splint body, fabricated of a resilient material, is disclosed. This splint body, however, presents some resistance to movements even in an "allowed" direction and allows some movement, albeit against resistance, in "forbidden" directions.

Another prior art example is US 5,232,136 which discloses an extension block finger splint for treating proximal interphalangeal joint injuries is disclosed. The finger splint comprises a body including a first portion located distally of the proximal interphalangeal joint, the first portion comprising a curved member dorsally orientable about the distal interphalangeal joint. The body further includes a second portion hingedly and resiliently connected to the first portion at a predetermined and, the second portion located proximally of the proximal interphalangeal joint and comprising a curved member dorsally orientable about the finger adjacent the proximal interphalangeal joint. A mechanism, removably disposed on the body, is provided for increasing the predetermined angle by a predetermined increment. Further, a mechanism is provided for releasably securing the splint to the finger. It appears desirable to provide a movement restraining device that presents no resistance to a movement in allowed directions and hard resistance to a movement in a forbidden direction. Under certain circumstances it is further often desirable to indicate to a nurse or to a health worker if the patient has made an attempt to move the restrained body part in a "forbidden" direction or beyond an allowed position, because even an attempt may put the patient's health at risk. Since a nurse cannot be next to the patient all the time, providing the nurse with an alarm immediately, or provide her upon return, or later upon demand, with information that the patient has moved the restrained body part into a certain position appears desirable.

The present invention attempts to address these needs. It provides a movement restraining device for partially immobilizing a body joint as set out in claim 1.

The movement restraining device can be attached to the skin at a body joint via the skin attachment means. The restraining device and the body joint are thereby restricted to pivoting in the same pivot direction, by approximately the same angle and approximately parallel to each other. The limitations imposed by the abutment surface and the abutting surface on the pivoting of the second stopper element relative to the first stopper element cause a limitation of the rotation of the body part about the joint axis up to an abutment. On the other hand, the free pivoting of the restraining device from the abutting pivot position in the second pivot direction facilitates a free, unimpeded rotation of the body part about the joint axis in the second, opposite pivot direction. Partial immobilization of the joint is thus achieved by the restraining device, attached to the body joint, allowing free movement up to the abutting pivot position, and creating an abutment to block further movement beyond the abutting pivot position.

The electrical contact elements are brought into contact with each other when the second stopper element is pivoted into the abutting pivot position. When the electrical contact is established, a current may flow through the contact elements, and an indicator device, e.g. an LED or a buzzer or a transmitter, may be powered to indicate to the nurse or to the patient that the abutting pivot position has been achieved at least once, i.e. that the patient has attempted at least once to move the restrained body part into, and possibly beyond, the abutting pivot position.

In this disclosure, the words "electrical contact element" are to be understood in a broad sense. Specifically, the electrical contact elements do not need to be mechanically attached to stopper elements. For example, if the abutting pivot position is sensed by a sensor like e.g. a proximity sensor, and the sensor signal causes a transistor to become conductive, the electrical contacts to which the source and the drain of the transistor are connected are considered to be brought into electrical contact with each other when the second stopper element is pivoted into the abutting pivot position.

A movement restraining device according to the invention may be used to partially immobilize body joints like, for example, joints at a human wrist or elbow, a human ankle or knee, a human finger or toe. Similarly, it may for example be used for corresponding body joints of animals like horses, cats, dogs, horses, or the like. Generally, a movement restraining device according to the invention provides particular benefits when used to partially immobilize body joints that facilitate rotation of a body part about a joint axis. In particular, the rotation of the body part may be a pivoting rotation of a body part. Hence the body joint may be a pivoting joint. The pivoting rotation of a body part may be a pivoting rotation of a body part with respect to another body part. An example of such a pivoting rotation is the pivoting rotation of the forearm about an elbow axis with respect to the upper arm.

The first stopper element may have the shape of a brick or of a cube or of a prism, specifically of a triangular prism. It may be made of, or comprise, a polymeric material. The first stopper element may be formed as a single piece. It may comprise outer surfaces forming a hollow space between them.

The second stopper element may have the shape of a brick or of a cube. It may be made of, or comprise, a polymeric material. The second stopper element may be formed as a single piece. It may comprise outer surfaces forming a hollow space between them. Independent of its shape, a stopper element (the first, the second or any further stopper element) may be hollow or solid. It may be a single piece or comprise a plurality of pieces.

The first stopper element and the second stopper element may be adapted to be engaged with each other, or may be engaged with each other. The first stopper element may comprise a recess for receiving a protrusion of the second stopper element. The second stopper element may comprise a protrusion for engagement with the first stopper element. Vice versa, the second stopper element may comprise a recess for receiving a protrusion of the first stopper element. The first stopper element may comprise a protrusion for engagement with the second stopper element.

Alternatively, the first stopper element and the second stopper element are not engaged with each other and/or are not adapted to be engaged with each other.

An abutment surface, e.g. the abutment surface of the first stopper element, or an abutment surface of the second, of a third, fourth or of a further stopper element, may be an outer surface, or a portion of an outer surface, of the stopper element. An abutment surface may be flat or comprise a flat portion. Alternatively, an abutment surface may be curved or comprise a curved portion. A curved abutment surface may be curved in one direction, i.e. it may comprise a two-dimensional curvature. An example of a two-dimensional curvature is the curved surface of a cylinder. A curved abutment surface may be curved in two directions, i.e. it may comprise a three-dimensional curvature. An example of a three-dimensional curvature is the surface of a sphere.

The second stopper element may also comprise an abutment surface, the "second abutment surface". Two or more or all abutment surfaces of a restraining device according to the invention, may be oriented, in the abutting pivot position, parallel to each other. They may be oriented parallel to each other within a +/-10-degree angle interval.

The abutment surface of the first stopper element and the abutting surface may be parallel and directly adjacent to each other in the abutting pivot position of the second stopper element.

An abutting surface of a stopper element, e.g. the abutting surface of the second stopper element, may comprise raised surface features that match with corresponding recessed surface features comprised in the abutment surface of an adjacent stopper element, when the stopper element is in the abutting pivot position relative to the adjacent stopper element. This may help to define the abutting pivot position more precisely.

In the context of this disclosure, an abutting surface is understood to be in contact with an abutment surface if the two surfaces touch each other in more than one point or along more than one line, i.e. when there is an extended contact area, or in other words, a surface contact, between the two surfaces. For the avoidance of doubt, two flat surfaces are not considered to be in contact when they have a common edge, but are not parallel to each other.

The first stopper element may comprise a first skin-facing surface. The second stopper element may comprise a second skin-facing surface. The skin attachment means may be suitable for attaching the first and the second stopper element to skin at the body joint such that the first skin-facing surface and the second skin-facing surface are oriented towards the skin. The presence of a skin-facing surface may provide a suitable area for attaching the stopper elements to skin reliably. A skin-facing surface may be adapted, e.g. in size, surface texture, or shape, for attaching the stopper elements to skin. This may facilitate reliable attachment to skin and consistent orientation of a stopper element relative to the skin and the body joint.

A skin-facing surface, i.e. the first and/or the second skin-facing surface, and/or a skin-facing surface of a third, fourth and/or generally of a further stopper element, may be an outer surface, or a portion of an outer surface, of the respective stopper element. A skin-facing surface may be flat or comprise a flat portion. Alternatively, a skin-facing surface may be curved or comprise a curved portion. A curved skin-facing surface may be curved in one direction, i.e. it may comprise a two-dimensional curvature. A curved skin-facing surface may be curved in two directions, i.e. it may comprise a three-dimensional curvature. A curved skin-facing surface may be shaped to follow the contour of the skin at the body joint, so that the restraining device may be more comfortable to wear and/or it may be easier to attach the stopper element to the skin at the body joint.

The second stopper element is arranged adjacent to the first stopper element, in an adjacency direction. The adjacency direction is defined by the arrangement of the second stopper element relative to the first stopper element. An adjacency direction may be the direction of a vector connecting a point on a surface of the first stopper element with a point on a surface of the second stopper element.

If the first and the second stopper element have identical outer shapes, a vector from the geometric centre of the first stopper element to the geometric centre of the second stopper element defines the adjacency direction. If the first and the second stopper element have identical outer shapes, a vector from a specific feature of the first stopper element to the corresponding specific feature of the second stopper element may define the adjacency direction. Generally, where two or more stopper elements are arranged adjacent to each other in a straight line or chain, the direction of this line is an adjacency direction. Most generally, an adjacency direction may be defined by a vector connecting any point in the first stopper element to any point in the second stopper element.

Two stopper elements of a movement restraining device are considered adjacent stopper elements if they are arranged next to each other such that no other stopper element is arranged between them.

However, adjacent stopper elements may be separated by a gap. The gap may be narrow. It may, for example, be smaller than 1mm, 2mm, or 5mm, as measured in an adjacency direction. The first stopper element and the second stopper element may be separated by a gap when the second stopper element is in a pivot position different from the abutting pivot position.

The second stopper element is pivotable relative to the first stopper element about a virtual pivot axis. The pivot axis may extend perpendicularly to the adjacency direction. The term "pivotable", in the context of the present disclosure, implies rotation about a single axis. A first pivot direction may be an angular direction of rotation about the pivot axis, e.g. clockwise, when viewed from a certain point. An opposite pivot direction may then be the opposite angular direction of rotation about the same axis, e.g. anticlockwise, when viewed from the same viewpoint. The second stopper element is pivotable between at least the abutting pivot position and a non-abutting pivot position. The second stopper element can be brought, i.e. pivoted, into the abutting pivot position, i.e. it can be brought, i.e. pivoted, from a non-abutting pivot position into the abutting pivot position.

Turning to the pivot axis and its orientations, the pivot axis is a mathematical, i.e. virtual, line about which the second stopper element can rotate or pivot relative to the first stopper element. The pivot axis, in particular its position and orientation, may be defined by a physical element, e.g. by a hinge axis or a pin. The virtual pivot axis may be defined, for example, by a recess comprised in the first stopper element. The recess may be adapted to receive, or may receive, a matching protrusion comprised in the second stopper element, so that the second stopper element may be pivotably engaged with the first stopper element.

Where the stopper elements have skin-facing surfaces, the pivot axis may be defined by a flexible adhesive tape connecting the first stopper element and the second stopper element. A first portion of the tape may be attached to the first skin-facing surface, a second portion of the tape may be applied to the second skin-facing surface. A third portion, or generally a portion, of the tape may form a hinge and a hinge axis, about which the second stopper element is pivotable relative to the first stopper element. Therefore, more generally, the pivot axis may be defined by the skin attachment means or a portion of the skin attachment means.

The pivot axis may be defined by an axis of a hinge arranged between the first stopper element and the second stopper element.

The pivot axis may extend in a direction perpendicularly to the adjacency direction. The pivot axis may alternatively extend in a direction parallel to the adjacency direction.

Where the first stopper element has a rectangular side face, i.e. a rectangle-shaped surface portion facing the second stopper element, the pivot axis may extend parallel to an edge of the rectangle. This orientation of the pivot axis may facilitate particularly space-saving pivoting.

Where a first edge of the side face rectangle extends parallel to a skin-facing surface of the first stopper element, the pivot axis may extend parallel to the first edge. This arrangement may allow for pivoting the second stopper element away from or towards the skin before being attached to the skin.

Alternatively, where a second edge of the side face rectangle extends perpendicular to the skin-facing surface, the pivot axis may extend parallel to the second edge. This arrangement may allow for pivoting the second stopper element parallel to the skin.

Where the movement restraining device comprises a third stopper element, the third stopper element may be arranged pivotably relative to the second stopper element as described above for the second stopper element. In particular, a virtual pivot axis of the third stopper element may be arranged parallel to the virtual pivot axis of the second stopper element. This allows the second and the third stopper elements to pivot in the same pivot direction, which is advantageous in that it allows movement of the joint, to which the restraining device is attached, in the pivot direction.

Generally, for a restraining device comprising a third stopper element and further stopper elements, each adjacent to another stopper element in the adjacency direction of the second stopper element, these stopper elements may be pivotable, relative to an adjacent stopper element, such that the respective pivot axes of all these stopper elements are oriented parallel to the pivot axis of the second stopper element.

Where the first stopper element comprises a first skin-facing surface, the pivot axis may be oriented parallel to the first skin-facing surface. Where the second stopper element comprises a second skin-facing surface, the parallel orientation of the pivot axis may allow for pivoting of the second stopper element, relative to the first stopper element, such that its skin-facing surface (the second skin-facing surface) pivots out of the plane of the first skin-facing surface. This may make the device particularly well suited for application to the inner bend of a hinge-type joint, e.g. inside an elbow joint.

Alternatively, the pivot axis may be oriented perpendicular to the first skin-facing surface. This orientation of the pivot axis may allow for pivoting of the second stopper element, relative to the first stopper element, such that its skin-facing surface (the second skin-facing surface) pivots "sideways" in the plane of the first skin-facing surface. This may make the device particularly well suited for application to a lateral side of a hinge-type joint, e.g. at the side of an elbow joint.

The movement restraining device of the present disclosure comprises skin attachment means for attaching the first and second stopper elements to the skin at the body joint which is to be partially immobilized. The word skin is to be understood, in the context of this disclosure, to describe the outer surface of the body, i.e. it encompasses bare skin, hairy skin, furred skin, and such skin of humans and animals.

Generally, stopper elements can be attached to skin in various ways. Skin attachment means may thus, for example, comprise a belt member for pulling around the stopper element and the body part to which the stopper element is to be attached.

Generally, a stopper element may be attached to skin directly or indirectly, i.e. with intermediate layers being arranged between the stopper element and the skin.

The skin attachment means may comprise a layer of adhesive, e.g. a layer of pressure-sensitive adhesive. The layer of adhesive may be arranged on an outer surface of the stopper element. In particular, it may be arranged on a skin-facing surface, e.g. on the first skin-facing surface and/or on the second skin-facing surface, and/or on the skin-facing surfaces of all stopper elements of the restraining device. Specifically, the skin attachment means may comprise a layer of pressure-sensitive adhesive arranged on at least a portion of a skin-facing surface. The layer of adhesive may be continuous or patterned.

Generally, attachment of a movement restraining device to skin via adhesive may provide for attachment over an extended area of the skin, thus distributing mechanical stress over a large skin area. This may avoid injuries or bruises on the skin. Also, attachment via adhesive may be a space-saving way of attaching a movement restraining device to the body of a patient or an animal. Adhesive attachment may also be a cost-effective means of attachment, in particular for one-time, single-use movement restraining devices.

The adhesive may be a skin-friendly adhesive.

The skin attachment means in a device according to the present invention may comprise a carrier tape. The carrier tape has a first and an opposed second major surface. It may comprise, for example, a polymeric material or a woven or a nonwoven fabric. The carrier tape may comprise mechanical fastening means like hook or loop material, or other mechanical fastening means. The first stopper element and/or the second stopper element may be attached to the carrier tape via these mechanical fastening means.

The skin attachment means may comprise a carrier tape and pressure-sensitive adhesive, arranged on one major surface of the carrier tape. The pressure-sensitive adhesive may be suitable for attaching the carrier tape to the skin. The skin attachment means may comprise a carrier tape having two opposed major surfaces, wherein the adhesive is arranged on one of the major surfaces.

The first stopper element may be attached to the second, opposed major surface of the carrier tape. Specifically, the first skin-facing surface of the first stopper element may be attached to the second, opposed major surface of the carrier tape. The first stopper element can thereby be attached to the skin via the carrier tape and the adhesive.

Generally, the first and the second stopper element may be attached to the second, opposed major surface of the carrier tape. The first skin-facing surface of the first stopper element and the second skin-facing surface of the second stopper element may be attached to the second, opposed major surface of the carrier tape. Generally, for movement restraining devices having first, second and further stopper elements, the skin-facing surfaces of all stopper elements may be attached to the carrier tape.

The carrier tape may be flexible or bendable. A flexible or bendable carrier tape may provide for a connection between the first stopper element and the second stopper element. Specifically, a bendable or flexible carrier tape may provide for a pivotable connection between the stopper elements.

The skin attachment means may comprise a first and a second carrier tape. The first stopper element may be attached to skin via the first carrier tape. The second stopper element may be attached to skin via the second carrier tape. Specifically, the first skin-facing surface of the first stopper element may be attached to skin via the first carrier tape. The second skin-facing surface of the second stopper element may be attached to skin via the second carrier tape.

The first stopper element and the second stopper element may be attached to each other by the skin attachment means. Where the skin attachment means comprises a carrier tape, they may be attached to each other by the carrier tape. The stopper elements may be attached to each other such that the second stopper element is pivotable with respect to the first stopper element. Generally, all stopper elements may be attached to each other by the skin attachment means. Such an attachment between all stopper elements may provide for all components of the movement restraining device being connected with each other, which may facilitate handling of the device or its application to the skin.

A movement restraining device according to the present disclosure may comprise a plurality of further stopper elements, each comprising an abutment surface, an abutting surface, and a skin-facing surface. Each of the further stopper elements may be arranged, in the adjacency direction, adjacent to the first stopper element or to the second stopper element or to one of the further stopper elements. Each further stopper element may be pivotable, relative to an adjacent stopper element, about a virtual pivot axis. The pivot axis may, for example, extend perpendicularly to the adjacency direction. Each of the further stopper elements may comprise skin attachment means for attaching the further stopper element to skin at the body joint to be partially immobilized in a way, that the skin-facing surface of the further stopper element is oriented towards the skin. The abutment surface of each further stopper element may be arranged and/or shaped such that, in an abutting pivot position, the abutment surface of the further stopper element is in contact with the abutting surface of the adjacent stopper element, thereby preventing pivoting in a first pivot direction, while allowing pivoting in a second pivot direction, opposite to the first pivot direction.

A movement restraining device as described here may thus comprise a chain of stopper elements, one adjacent to another in the adjacency direction defined by the arrangement of the first and the stopper element. Each of the further stopper elements may be pivotable with respect to an adjacent stopper element. This may make the chain deformable, so that it can change its form, e.g. from straight to curved, thereby following the movement of the body joint at which the device is attached to skin. The individual stopper elements can be pivoted relative to the adjacent stopper element up to individual abutting pivot positions, defined by position, shape and orientation of the abutment surface of one stopper element relative to the abutting surface of the adjacent stopper element. Thereby the change of the form of the entire chain of stopper elements is limited. The movement restraining device as a whole thus provides an abutment position for the entire device, which is defined by the combined effect of the abutting pivot positions of the individual stopper elements with respect to their adjacent stopper elements. Because the entire device is attached to the skin at the body joint, the abutment position of the entire device defines an abutment for the rotation of a body part rotating about the axis of the body joint.

For a given length of the movement restraining device, i.e. its extension in the adjacency direction, more but smaller stopper elements may allow for the device to follow a body contour more closely at the body joint. This may benefit adhesion to skin and comfort of the patient.

Generally, partial immobilization of a smaller body joint, e.g. of a finger joint, may require smaller stopper elements, because there is little space to attach stopper elements to skin. However, more reliable attachment of the movement restraining device to skin may generally require a larger surface of the skin attachment means, which may be achieved by having larger stopper elements, and in particular larger skin-facing surfaces of the stopper elements. Alternatively, skin attachment means may extend beyond the skin-facing surface of the first or the second stopper element. Generally, the skin attachment means may extend beyond the skin-facing surfaces of all stopper elements. This may help make the attachment of stopper elements to skin more reliable.

The first stopper element may be a single piece. The second stopper element may be a single piece. Each of the further stopper elements may be a single piece. In particular, the skin-facing surface, the abutting surface and the abutment surface of any stopper element may be surfaces of a single piece. "Single piece" is to be understood, in the context of this disclosure, as having the sense of "formed from a single piece of material", as opposed to elements that are assembled from two or more components.

Electrical contact elements, or briefly contact elements, may be electrically conductive elements as they are widely known. At least a portion of their outer surface is electrically conductive. Examples include metallic contact elements or metallized, i.e. metal-coated, contact elements, or contact elements comprising electrically conductive polymers. An electrical contact element may be a conductive coating or a conductive layer, e.g. on a polymeric, electrically insulating film. An electrical contact element may be a conductive coating on the abutment surface of the first stopper element or of any other stopper element. An electrical contact element may be a conductive coating on the abutting surface of the second stopper element or of any other stopper element.

An electrical contact element may be comprised in a stopper element, e.g. in the first stopper element or in the second stopper element. It may be a conductive portion of a stopper element, e.g. of the first stopper element or of the second stopper element.

An electrical contact element may be resilient or it may comprise a resilient portion. An electrical contact element may be, for example, a metallic spring contact.

Electrical contact elements may be, for example, contact elements of a switch. The switch may be closed by pivoting the second stopper element into the abutting pivot position. The first and the second electrical contact element may be, for example, contacts of a transistor, e.g. the source contact and the drain contact. The transistor may be comprised in an electrical circuit adapted to make the transistor conductive when the second stopper element is pivoted into the abutting pivot position. The transistor then brings the first contact element into electrical contact with the second contact element.

In a movement restraining device according to the present invention, the first contact element may be comprised in the first stopper element, e.g. in an outer surface of the first stopper element. The first contact element may be comprised in the abutment surface or arranged on the abutment surface of the first stopper element.

The second contact element may be comprised in the second stopper element, e.g. in an outer surface of the second stopper element. The second contact element may be comprised in the abutting surface of the second stopper element or arranged on the abutting surface.

Generally, the first and the second contact elements may be comprised in different stopper elements. They may be comprised in different adjacent stopper elements.

Arrangement in different stopper elements may make assembly of a movement restraining device particularly cost-effective, because no separate support for contact elements may be required, and the surface contact in the abutting pivot position may be used effectively to bring the electrical contact elements into mechanical and electrical contact. Arrangement of a contact element in or on an abutting surface or abutment surface may be a particularly cost-effective way of building a movement-restraining device according to the invention, because the abutment/abutting surface can be used for two purposes simultaneously, namely providing mechanical abutment and electrical contact.

In certain devices according to the present invention, the first contact element and the second contact element may be comprised in the same stopper element, e.g. in the first or in the second stopper element. This may be beneficial in that all contact elements and potentially all other electrical elements like a battery or an indicator device, can be placed in one single stopper element, so that all other stopper elements can remain free of electrical elements. Such a concentration of electrical elements in one stopper element may make assembly of the device more cost-effective.

Where the first contact element and the second contact element are comprised in the same stopper element, an adjacent stopper element may be adapted to push, when pivoting into the abutting pivot position, one contact element towards the other contact element and thereby bring the contact elements into mechanical and electrical contact with each other.

In a movement restraining device according to the present invention, the first contact element can be brought into electrical contact with the second contact element by pivoting the second stopper element into the abutting pivot position. This is meant to imply, in the context of this disclosure, that the first contact element is brought from non-contact (in any other pivot position) into contact (in the abutting pivot position).

Generally, the first contact element may not be in electrical contact with the second contact element when the second stopper element is not in the abutting pivot position. The first contact element may be in electrical contact with the second contact element only when the second stopper element is in the abutting pivot position. Such movement restraining devices may facilitate an indication that the patient has moved the body joint, to which the device is attached, into a "limit" position, from which onwards he may not move it any further. The limit position of the joint corresponds to the abutting pivot position of the movement restraining device, which is attached to skin at the joint.

The first and/or the second contact element may comprise an electrically conductive polymer or an electrically conductive polymeric film or a metallized polymeric film or a metal film or an electrically conductive coating. The first and/or the second contact element may comprise an electrically conductive polymeric film or a metallized polymeric film or a metal film or an electrically conductive coating, any of these being comprised in the first or in the second stopper element. Contact elements comprising a polymeric film or a metallized polymeric film or a metal film or an electrically conductive coating may be comparatively thin and may hence increase the size or volume of the device only to a negligible degree. Metallized layers or conductive coatings often stick strongly to a surface on which they are applied, and are hard to remove, so that the entire device may end up being more reliable and more durable.

A movement restraining device according to one aspect of the invention may comprise an indicator device. The indicator device may be an electrical indicator device. It may be electrically connected to the first contact element or to the second contact element or to both contact elements. It may be operable to provide a visual, audible or electronic indication when the contact elements are in electrical contact with each other. The indicator device may be operable to transmit a visual, audible or electronic indication when the contact elements are in electrical contact with each other. The indicator device may comprise storage means for storing the visual, audible or electronic indication.

The presence of an indicator device may be useful for notifying other persons, e.g. health workers or nurses, in an unmistakable manner that the patient has moved the body joint such that the second stopper element of the movement restraining device was brought into the abutting pivot position. The indicator device may be adapted to provide an indication even after the patient has moved the second stopper element back out of the abutting pivot position after having brought it into the abutting pivot position by moving the partially restrained body part.

The indicator device may comprise a light source for providing a visual indication. The light source may be, for example, a light-emitting diode ("LED") or an incandescent light source. The light source may be the light source of a display of a digital device, on which the indication is displayed, e.g. in text, by graphics or via an icon. Light sources are effective and cost-effective means to attract the attention of a person.

The indicator device may comprise a loudspeaker for providing an audible indication. The loudspeaker may be a buzzer. Loudspeakers are effective and cost-effective means to attract the attention of a person. A movement restraining device in which the indicator device comprises a loudspeaker may be particularly suitable to indicate to the patient that he is moving the body part into a "forbidden" position, allowing the patient to interrupt that movement immediately.

The indicator device may comprise an antenna for providing an electronic indication. In particular, the indicator device may comprise an RFID tag or an RFID reader for providing an electronic indication. Generally, the antenna may be adapted to emit or receive electromagnetic waves. The antenna may be an antenna for wireless communication, in particular for digital wireless communication like, for example, communication via mobile telephones, wireless LAN, Bluetooth, or similar. The indication may be an indication transmitted via wireless communication. The antenna may transmit an electromagnetic signal which codes information comprising the indication. An electronic indication may be advantageous in that it may be transmitted over longer distances. Digital indications may be received and/or recorded automatically by computer systems used in hospitals.

Generally, the indicator device may be arranged outside the movement restraining device. In that case it may be connectable or connected with one or both of the electrical contact elements.

Alternatively, and independent of other features, the indicator device may be comprised in the movement restraining device. It may be arranged inside a cavity formed by the first or the second stopper element, or inside a cavity formed by a further stopper element. Such a cavity may be formed by two, three of four side walls of a stopper element. The cavity may comprise two, three or four walls. The cavity may be partially open.

A cavity may be advantageous in that it may protect the indicator device against environmental impacts. A partially open cavity may allow for manual access to the indicator device, e.g. for exchanging the indicator device or for changing a battery.

It may be desirable to use the movement restraining device more than once. In particular it may be desired to use it on the same patient multiple times, or on different patients. The movement restraining device may therefore be adapted to be sterilisable, e.g. adapted by the choice of materials it is made from, or by coating its surfaces. In particular, the contact elements may be adapted to be sterilisable, e.g. adapted by the choice of materials they are made from, or by coating of their surfaces. The movement restraining device or the contact elements may be sterilisable, for example, by electron beam methods, by gamma ray methods, or by methods using ethylene oxide.

The invention will now be described in more detail with reference to the following Figures exemplifying particular embodiments of the invention. Certain dimensions may be shown exaggerated for greater clarity.
- Fig. 1: Side view of a first movement restraining device according to the invention;
- Fig. 2: Side view of the first movement restraining device in an abutting pivot position;
- Fig. 3: Perspective view of a second movement restraining device according to the invention; and
- Fig. 4: Perspective view of a third movement restraining device according to the invention.

Herein below various embodiments of the present invention are described and shown in the drawings wherein like elements are provided with the same reference numbers.

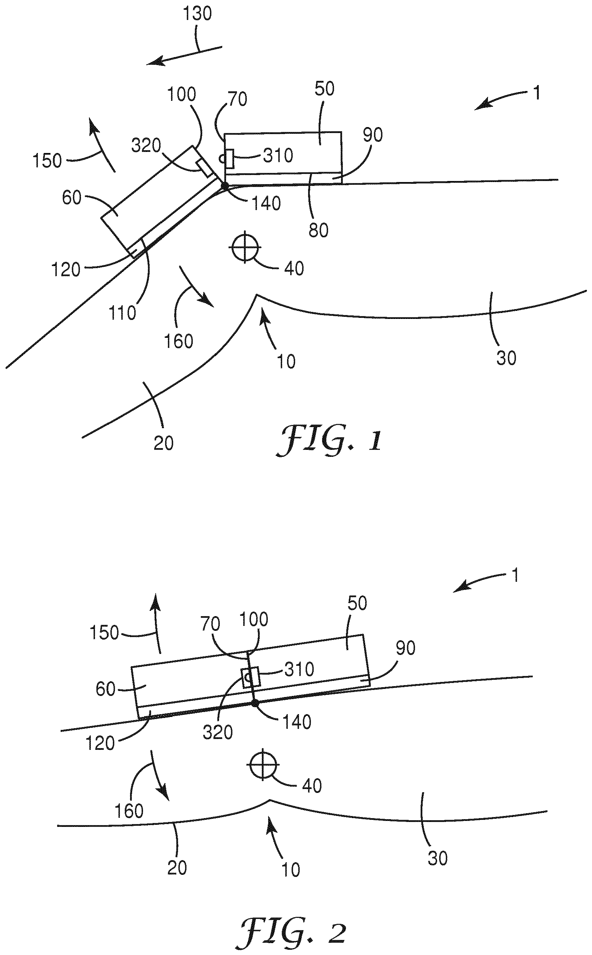

**Figure 1** is an idealized side view of a first movement restraining device 1, attached to skin at a body joint 10 of a human patient or of an animal. The body joint 10 facilitates rotation of a first body part 20, relative to a second body part 30, about a joint axis 40, which is oriented perpendicularly to the plane of the drawing.

The movement restraining device 1 comprises a first stopper element 50 and a second stopper element 60. Both stopper elements 50, 60 have identical outer shapes, namely the shape of a brick, of which a side surface is visible in the Figure. The first stopper element 50 comprises a flat abutment surface 70 and a first skin-facing surface 80. The abutment surface 70 is oriented perpendicularly to the first skin-facing surface 80, and in the plane of the drawing their surface normals form an angle of 90° relative to each other. The first stopper element 50 is attached to the skin via a first adhesive layer 90, which is arranged on the first skin-facing surface 80. The first adhesive layer 90 is a layer of a skin-friendly pressure-sensitive adhesive.

The second stopper element 60 comprises an abutting surface 100 and a second skin-facing surface 110. The second stopper element 60 is attached to the skin via a second adhesive layer 120, arranged on the second skin-facing surface 110. The second stopper element 60 is arranged adjacent to the first stopper element 50. The adjacency direction is indicated by arrow 130. It extends in the plane of the drawing. The adjacency direction 130 indicates a direction which points generally from the first stopper element 50 towards the second stopper element 60.

The second stopper element 60 is pivotable relative to the first stopper element 50, about a virtual pivot axis 140, which extends perpendicularly to the plane of the drawing and parallel to the first skin-facing surface 80. The virtual pivot axis 140 is perpendicular, i.e. orthogonal, to the adjacency direction 130.

The first stopper element 50 comprises a first electrical contact element 310, and the second stopper element 60 comprises a second electrical contact element 320. The contact elements 310, 320 are electrically conductive metal sheets, arranged on side walls of the respective stopper elements 50, 60. The first contact element 310 comprises a protrusion which extends beyond the first stopper element 50 towards the second stopper element 60. The contact elements 310, 320 are connected to wires, which are not shown.

In Figure 1, the movement restraining device 1 is shown in a pivot position which is not an abutting pivot position. The first contact element 310 is not in electrical contact with the second contact element 320. In the pivot position shown in Figure 1, the first body part 20 can be freely rotated, relative to the second body part 30, about the joint axis 40 in a first pivot direction, indicated by arrow 150, i.e. clockwise, or in a second pivot direction, indicated by arrow 160, opposite to the first pivot direction 150, i.e. anti-clockwise (in the drawing). In the pivot position shown in Figure 1, the movement of the body joint 10 is not impeded. Because the movement restraining device 1 is attached to the skin and thus pivots about the pivot axis 140 as the first body part 20 rotates about the joint axis 40, the anti-clockwise rotation of the first body part 20 is limited by the abutting surface 100 of the second stopper element 60 abutting against the abutment surface 70 of the first stopper element 50 in an abutting pivot position. This is shown in Figure 2.

**Figure 2** is an idealized side view of the first movement restraining device 1, attached to skin at the body joint 10. However, the device 1 is shown in the abutting pivot position, in which the abutting surface 100 of the second stopper element 60 is in contact with the abutment surface 70 of the first stopper element 50. The device 1 has been brought into this abutting pivot position by pivoting the second stopper element 60, relative to the first stopper element 50, about the pivot axis 140. In this abutting pivot position, the first contact element 310 is in electrical contact with the second contact element 320. The protrusion of the first contact element 310 is in resilient mechanical and hence electrical contact with a surface of the second contact element 320.

The abutment of the abutting surface 100 against the abutment surface 70 prevents pivoting, or further pivoting, in the first pivot direction 150. Due to the device 1 being attached to skin, the movement restraining device 1 prevents rotation, i.e. further rotation, of the first body part 20 about the joint axis 40 in clockwise direction (in the drawing) beyond the position shown in Figure 2.

This mechanism provides partial immobilization of the body joint 10. The immobilization is partial, because the first body part 20 can be rotated freely in the anti-clockwise direction, while it can rotate in a clockwise direction only up to the abutment provided by the contact of the abutment surface 70 and the abutting surface 100 in the abutting pivot position. This helps avoid overstretching of the body joint 10 beyond the almost-straight position shown in Figure 2.

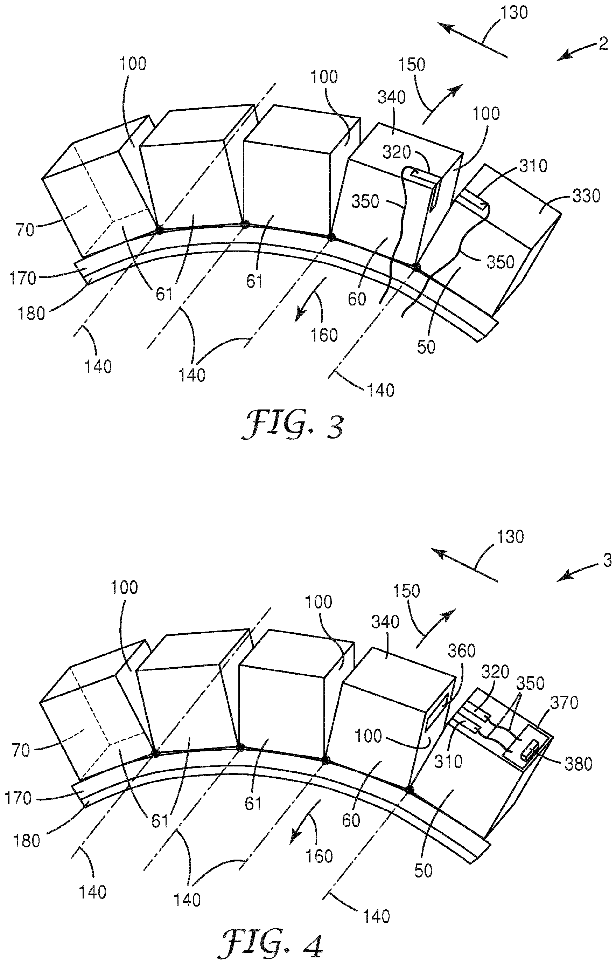

A movement restraining device according to the present disclosure may comprise more than two stopper elements 50, 60. An example of such a movement restraining device is shown in **Figure 3****,** in which a second movement restraining device 2 is shown in perspective view. It comprises, in addition to a first stopper element 50 and a second stopper element 60, three further stopper elements 61. Each of the stopper elements 50, 60, 61 has the shape of a cube and comprises an abutment surface 70 and an abutting surface 100, and a skin-facing surface (not visible in Figure 3). The stopper elements 50, 60, 61 are arranged adjacent to a neighbouring stopper element in an adjacency direction 130. In other words, the stopper elements 50, 60, 61 form a chain of stopper elements, one adjacent to another in the adjacency direction 130 defined by the arrangement of the first stopper element 50 and the second stopper element 60.

Each of the stopper elements 50, 60, 61 is pivotable, relative to the neighbouring stopper element 50, 60, 61 about a virtual pivot axis 140. The pivot axis 140 are parallel to each other and extend perpendicularly to the adjacency direction 130.

The first stopper element 50 comprises a first contact element 310, namely a strip of metallized adhesive tape, which is applied on the top surface 330 and on the abutment surface 70 of the first stopper element 50. The abutment surface 70 and the portion of the first contact element 310 on that surface 70 are not visible in Figure 3.

The second stopper element 60 comprises a second contact element 320, which is also a strip of metallized adhesive tape, applied on the top surface 340 and on the abutting surface 100 of the second stopper element 60. When the second stopper element 60 is pivoted from the non-abutting pivot position shown in Figure 3 into an abutting pivot position, the abutting surface 100 is in surface contact with the abutment surface 70. By pivoting the second stopper element 60 into the abutting pivot position, the first contact element 310 is brought into electrical contact with the second contact element 320. The metallized adhesive tape of the contact elements 310, 320 is sufficiently thin and sufficiently small to not prevent the surface contact between the abutting surface 100 of the second stopper element 60 and the abutment surface 70 of the first stopper element 50 in the abutting pivot position.

Two wires 350 are attached to the first contact element 310 and to the second contact element 320, respectively, which can be used for connecting the contact elements 310, 320 to an indicator device (not shown), which detects when the contact elements 310, 320 are in electrical contact and provides an indication to care professionals and to the patient.

The device 2 comprises skin attachment means in the form of a flexible carrier tape 170 and an adhesive layer 180. The carrier tape 170 and the adhesive layer 180 can be bent such as to allow pivoting of the stopper elements 50, 60, 61 relative to their adjacent stopper elements 50, 60, 61. The carrier tape 170 is a continuous polymeric film. It is adhesively attached to the skin-facing surfaces of the stopper elements 50, 60, 61. The stopper elements 50, 60, 61 are thus attached to each other by the carrier tape 170. On the underside (in Figure 3) of the carrier tape 170 the adhesive layer 180 is arranged, through which the stopper elements 50, 60, 61 of the movement restraining device 2 can be attached to the skin such that their respective skin-facing surfaces face the skin.

The abutment surfaces 70 are arranged and shaped such that, in an abutting pivot position, the abutment surface 70 of a stopper element 50, 60, 61 is in contact with the abutting surface 100 of the adjacent stopper element 50, 60, 61, so that further pivoting in a first pivot direction 150 is prevented, while pivoting in the second pivot direction 160, opposite to the first pivot direction 150, is still possible.

For each pair of stopper element 50, 60, 61 and adjacent stopper element 50, 60, 61, a pivot axis 140 is defined by the carrier tape 170, about which pivot axis 140 one stopper element 50, 60, 61 can pivot relative to the adjacent stopper element 50, 60, 61. Similarly, each pair of stopper element and adjacent stopper element define, through their relative arrangement, orientation and through the shape of the abutting surface 100 and abutment surface 70, an individual abutting pivot position. Once attached to the skin at a body joint, the movement restraining device 2 limits the movement of the body joint when all pairs of adjacent stopper elements 50, 60, 61 are in their abutting pivot position.

**Figure 4** shows, in a perspective view, a third movement restraining device 3 according to the present disclosure. The device is shown in a non-abutting pivot-position, i.e. the abutting surface 100 of the second contact element 60 is not in surface contact with the abutting surface 70 (not visible) of the first stopper element. The third movement restraining device 3 is identical to the second movement restraining device 2 shown in Figure 3, except for the following differences.

In the third movement restraining device 3, both the first contact element 310 and the second contact element 320 are comprised in the first stopper element 50. When the second stopper element 60 is pivoted into the abutting pivot position, where the abutting surface 100 is in surface contact with the abutment surface 70 of the first stopper element 50, the first contact element 310 and the second contact element 320 are pressed against a conductive contact strip 360, consisting of a piece of metallized adhesive tape, applied suitably on the abutting surface 100 of the second stopper element 60. The contact strip 360 is then in electrical contact with both contact elements 310, 320. Through this contact strip 360, the first contact element 310 is brought into electrical contact with the second contact element 320.

The first stopper element 50 comprises an indicator device 370, to which the wires 350 are connected. The indicator device 370 is a printed circuit board (PCB) with electrical circuitry. The PCB 370 comprises a light source 380, namely a light-emitting diode (LED) 380. The indicator device 370 is powered by a battery which is not shown.

Before the second stopper element 60 is pivoted about pivot axis 140, relative to the first stopper element 50, into the abutting pivot position, i.e. when the second stopper element 60 is in the pivot position shown in Figure 4, the contact elements 310, 320 are not in contact with the contact strip 360 and with each other. Once the second stopper element 60 has been pivoted into the abutting pivot position, both contact elements 310, 320 are in contact with the contact strip 360 and thereby in contact with each other. This is similar to a switch being closed. The indicator device 370 has circuitry (not shown) which senses that the contact elements 310, 320 are in contact and in response powers the LED 380, which lights up and thereby indicates visually that the second stopper element 60 is in the abutting pivot position.

It is contemplated to arrange the indicator device 370 inside the first stopper element 50, e.g. inside a cavity formed by the side walls and the skin-facing surface of the first stopper element 50.

It is also contemplated that one or more or all of the further stopper elements 61 could be provided with pairs of contact elements like the first and the second contact elements 310, 320, with a contact strip like contact strip 360. All these contact elements could be connected by wires like wires 350 to the indicator device 370. The LED 380 might be powered only when the contact elements of two or more or all pairs of contact elements are in contact with each other.

## Claims

1. Movement restraining device (1, 2, 3) for partially immobilizing a body joint (10) facilitating rotation of a body part (20) about a joint axis (40), the movement restraining device comprising
a) a first stopper element (50), comprising an abutment surface (70);
b) a second stopper element (60),
- comprising an abutting surface (100);
- being arranged, in an adjacency direction (130), adjacent to the first stopper element;
- being pivotable, relative to the first stopper element, about a virtual pivot axis (140),
wherein the second stopper element can be pivoted into an abutting pivot position, in which position the abutting surface is in surface contact with the abutment surface, the surface contact preventing pivoting in a first pivot direction (150), while allowing pivoting in a second pivot direction (160), opposite to the first pivot direction;
c) skin attachment means (90, 170, 180) for attaching the first stopper element and the second stopper element to skin at the body joint;
charactered by
d) a first electrical contact element (310) and a second electrical contact element (320), at least one of which is connectable to an indicator device (370) for providing a visual, audible or electronic indication when the first contact element and the second contact element are in electrical contact with each other,
wherein the first contact element can be brought into electrical contact with the second contact element by pivoting the second stopper element into the abutting pivot position.

2. Movement restraining device according to claim 1, wherein the first contact element is comprised in the first stopper element, and wherein the second contact element is comprised in the second stopper element.

3. Movement restraining device according to claim 1, wherein the first contact element and the second contact element are comprised in the first stopper element or arranged on the first stopper element.

4. Movement restraining device according to any one of the preceding claims, wherein the first contact element is comprised in the abutment surface (70), or is arranged on the abutment surface.

5. Movement restraining device according to any one of claims 1, 2, or 4, wherein the second contact element is comprised in the abutting surface (100), or is arranged on the abutting surface.

6. Movement restraining device according to any one of the preceding claims, wherein the first contact element is not in electrical contact with the second contact element when the second stopper element is not in the abutting pivot position.

7. Movement restraining device according to any one of the preceding claims, wherein the first and/or the second contact element comprises an electrically conductive polymer or an electrically conductive polymeric film or a metallized polymeric film or a metal film or an electrically conductive coating.

8. Movement restraining device according to any one of the preceding claims, further comprising an indicator device (370), electrically connected to the first contact element or to the second contact element or to both contact elements, operable to provide a visual, audible or electronic indication when the contact elements are in electrical contact with each other.

9. Movement restraining device according to claim 8, wherein the indicator device comprises a light source (380) for providing a visual indication.

10. Movement restraining device according to claim 8, wherein the indicator device comprises a loudspeaker for providing an audible indication.

11. Movement restraining device according to claim 8, wherein the indicator device comprises an antenna for providing an electronic indication.

12. Movement restraining device according to any one of claims 8 to 11, wherein the indicator device is arranged inside a cavity formed by the first or the second stopper element.

13. Movement restraining device according to any one of the preceding claims, wherein the contact elements are adapted to be sterilisable by electron beam methods, gamma ray methods, or by methods using ethylene oxide.

14. Movement restraining device according to any one of the preceding claims, wherein the contact elements are contacts of a transistor.

## Patentansprüche

1. Bewegungsrückhaltevorrichtung (1, 2, 3) zum teilweisen Immobilisieren eines Körpergelenks (10), das eine Drehung eines Körperteils (20) um eine Gelenkachse (40) herum erleichtert, wobei die Bewegungsrückhaltevorrichtung umfasst
a) ein erstes Stopperelement (50), das eine Anlageoberfläche (70) umfasst;
b) ein zweites Stopperelement (60),
- das eine anliegende Oberfläche (100) umfasst;
- das in einer benachbarten Richtung (130) dem ersten Stopperelement benachbart angeordnet ist;
- das in Bezug auf das erste Stopperelement um eine virtuelle Schwenkachse (140) herum schwenkbar ist,
wobei das zweite Stopperelement in eine anliegende Schwenkposition geschwenkt werden kann, in welcher Position die anliegende Oberfläche mit der Anlageoberfläche in Kontakt steht, wobei der Oberflächenkontakt ein Schwenken in eine erste Schwenkrichtung (150) verhindert, während er ein Schwenken in eine zweite Schwenkrichtung (160), die der ersten Schwenkrichtung entgegengesetzt ist, ermöglicht;
c) Hautbefestigungsmittel (90, 170, 180) zum Befestigen des ersten Stopperelements und des zweiten Stopperelements an Haut an dem Körpergelenk; **gekennzeichnet durch**
d) ein erstes elektrisches Kontaktelement (310) und ein zweites elektrisches Kontaktelement (320), von denen mindestens eines mit einer Indikatorvorrichtung (370) zum Bereitstellen einer optischen, akustischen oder elektronischen Anzeige verbindbar ist, wenn das erste Kontaktelement und das zweite Kontaktelement miteinander in elektrischem Kontakt stehen,
wobei das erste Kontaktelement durch Schwenken des zweiten Stopperelements in die anliegende Schwenkposition mit dem zweiten Kontaktelement in elektrischen Kontakt gebracht werden kann.

2. Bewegungsrückhaltevorrichtung nach Anspruch 1, wobei das erste Kontaktelement in dem ersten Stopperelement enthalten ist, und wobei das zweite Kontaktelement in dem zweiten Stopperelement enthalten ist.

3. Bewegungsrückhaltevorrichtung nach Anspruch 1, wobei das erste Kontaktelement und das zweite Kontaktelement in dem ersten Stopperelement enthalten oder an dem ersten Stopperelement angeordnet sind.

4. Bewegungsrückhaltevorrichtung nach einem der vorstehenden Ansprüche, wobei das erste Kontaktelement in der Anlageoberfläche (70) enthalten ist oder an der Anlageoberfläche angeordnet ist.

5. Bewegungsrückhaltevorrichtung nach einem der Ansprüche 1, 2 oder 4, wobei das zweite Kontaktelement in der anliegenden Oberfläche (100) enthalten ist oder an der anliegenden Oberfläche angeordnet ist.

6. Bewegungsrückhaltevorrichtung nach einem der vorstehenden Ansprüche, wobei das erste Kontaktelement mit dem zweiten Kontaktelement nicht in elektrischem Kontakt steht, wenn sich das zweite Stopperelement nicht in der anliegenden Schwenkposition befindet.

7. Bewegungsrückhaltevorrichtung nach einem der vorstehenden Ansprüche, wobei das erste und/oder das zweite Kontaktelement ein elektrisch leitfähiges Polymer oder eine elektrisch leifähige Polymerfolie oder eine metallisierte Polymerfolie oder eine Metallfolie oder eine elektrisch leitfähige Beschichtung umfasst.

8. Bewegungsrückhaltevorrichtung nach einem der vorstehenden Ansprüche, ferner umfassend eine Anzeigevorrichtung (370), die mit dem ersten Kontaktelement oder mit dem zweiten Kontaktelement oder mit beiden Kontaktelementen elektrisch verbunden ist und dazu betreibbar ist, eine optische, akustische oder elektronische Anzeige bereitzustellen, wenn die Kontaktelemente miteinander in elektrischem Kontakt stehen.

9. Bewegungsrückhaltevorrichtung nach Anspruch 8, wobei die Anzeigevorrichtung eine Lichtquelle (380) zum Bereitstellen einer optischen Anzeige umfasst.

10. Bewegungsrückhaltevorrichtung nach Anspruch 8, wobei die Anzeigevorrichtung einen Lautsprecher zum Bereitstellen einer akustischen Anzeige umfasst.

11. Bewegungsrückhaltevorrichtung nach Anspruch 8, wobei die Anzeigevorrichtung eine Antenne zum Bereitstellen einer elektronischen Anzeige umfasst.

12. Bewegungsrückhaltevorrichtung nach einem der Ansprüche 8 bis 11, wobei die Anzeigevorrichtung innerhalb eines Hohlraums, der durch das erste oder das zweite Stopperelement gebildet wird, angeordnet ist.

13. Bewegungsrückhaltevorrichtung nach einem der vorstehenden Ansprüche, wobei die Kontaktelemente dazu ausgelegt sind, durch Elektronenstrahlverfahren, Gammastrahlverfahren oder durch Verfahren unter Verwendung von Ethylenoxid sterilisierbar zu sein.

14. Bewegungsrückhaltevorrichtung nach einem der vorstehenden Ansprüche, wobei die Kontaktelemente Kontakte eines Transistors sind.

## Revendications

1. Dispositif de restriction de mouvement (1, 2, 3) destiné à immobiliser partiellement une articulation du corps (10) facilitant la rotation d'une partie du corps (20) autour d'un axe d'articulation (40), le dispositif de restriction de mouvement comprenant
a) un premier élément d'arrêt (50), comprenant une surface de butée (70) ;
b) un deuxième élément d'arrêt (60),
- comprenant une surface venant en butée (100) ;
- étant agencé, dans une direction de contiguïté (130), adjacent au premier élément d'arrêt ;
- pouvant pivoter, par rapport au premier élément d'arrêt, autour d'un axe de pivotement virtuel (140),
dans lequel le deuxième élément d'arrêt peut pivoter dans une position de pivotement venant en butée, position dans laquelle la surface venant en butée est en contact de surface avec la surface de butée, le contact de surface empêchant le pivotement dans une première direction de pivotement (150), tout en permettant le pivotement dans une deuxième direction de pivotement (160), opposée à la première direction de pivotement ;
c) un moyen de fixation à la peau (90, 170, 180) destiné à fixer le premier élément d'arrêt et le deuxième élément d'arrêt à la peau au niveau de l'articulation du corps ;
**caractérisé par**
d) un premier élément de contact électrique (310) et un deuxième élément de contact électrique (320), dont au moins l'un peut être connecté à un dispositif indicateur (370) destiné à fournir une indication visuelle, audible ou électronique lorsque le premier élément de contact et le deuxième élément de contact sont en contact électrique l'un avec l'autre,
dans lequel le premier élément de contact peut être amené en contact électrique avec le deuxième élément de contact par pivotement du deuxième élément d'arrêt dans la position de pivotement venant en butée.

2. Dispositif de restriction de mouvement selon la revendication 1, dans lequel le premier élément de contact est compris dans le premier élément d'arrêt, et dans lequel le deuxième élément de contact est compris dans le deuxième élément d'arrêt.

3. Dispositif de restriction de mouvement selon la revendication 1, dans lequel le premier élément de contact et le deuxième élément de contact sont compris dans le premier élément d'arrêt ou agencés sur le premier élément d'arrêt.

4. Dispositif de restriction de mouvement selon l'une quelconque des revendications précédentes, dans lequel le premier élément de contact est compris dans la surface de butée (70), ou est agencé sur la surface de butée.

5. Dispositif de restriction de mouvement selon l'une quelconque des revendications 1, 2, ou 4, dans lequel le deuxième élément de contact est compris dans la surface venant en butée (100), ou est agencé sur la surface venant de butée.

6. Dispositif de restriction de mouvement selon l'une quelconque des revendications précédentes, dans lequel le premier élément de contact n'est pas en contact électrique avec le deuxième élément de contact lorsque le deuxième élément d'arrêt n'est pas dans la position de pivotement venant en butée.

7. Dispositif de restriction de mouvement selon l'une quelconque des revendications précédentes, dans lequel le premier et/ou le deuxième élément de contact comprend un polymère électriquement conducteur ou un film polymère électriquement conducteur ou un film polymère métallisé ou un film métallique ou un revêtement électriquement conducteur.

8. Dispositif de restriction de mouvement selon l'une quelconque des revendications précédentes, comprenant en outre un dispositif indicateur (370), connecté électriquement au premier élément de contact ou au deuxième élément de contact ou aux deux éléments de contact, pouvant fonctionner pour fournir une indication visuelle, audible ou électronique lorsque les éléments de contact sont en contact électrique l'un avec l'autre.

9. Dispositif de restriction de mouvement selon la revendication 8, dans lequel le dispositif indicateur comprend une source de lumière (380) destinée à fournir une indication visuelle.

10. Dispositif de restriction de mouvement selon la revendication 8, dans lequel le dispositif indicateur comprend un haut-parleur destiné à fournir une indication audible.

11. Dispositif de restriction de mouvement selon la revendication 8, dans lequel le dispositif indicateur comprend une antenne destinée à fournir une indication électronique.

12. Dispositif de restriction de mouvement selon l'une quelconque des revendications 8 à 11, dans lequel le dispositif indicateur est agencé à l'intérieur d'une cavité formée par le premier ou le deuxième élément d'arrêt.

13. Dispositif de restriction de mouvement selon l'une quelconque des revendications précédentes, dans lequel les éléments de contact sont adaptés pour être stérilisables par des procédés par faisceau d'électrons, des procédés par rayons gamma, ou par des procédés utilisant de l'oxyde d'éthylène.

14. Dispositif de restriction de mouvement selon l'une quelconque des revendications précédentes, dans lequel les éléments de contact sont des contacts d'un transistor.
